# EUROPÄISCHE PATENTSCHRIFT

(11) **EP 1 208 140 B1**
(45) Veröffentlichungstag und Bekanntmachung des Hinweises auf die Patenterteilung: **28.06.2006**
(21) Anmeldenummer: 00960450.5
(22) Anmeldetag: 14.08.2000
(51) Int. Cl.: C08J 9/28, A61K 9/20, A61K 9/16, C08L 5/08

(54) **SCHNELLZERFALLENDE PELLETS AUF DER BASIS VON CHITOSAN**
RAPIDLY DECOMPOSING CHITOSAN-BASED PELLETS
PASTILLES A DECOMPOSITION RAPIDE, A BASE DE CHITOSANE

(30) Priorität: 27.08.1999 DE 19940795
(43) Veröffentlichungstag der Anmeldung: 29.05.2002
(73) Patentinhaber: LTS LOHMANN Therapie-Systeme AG, 56626 Andernach (DE)
(72) Erfinder: HOFFMANN, Hans-Rainer, 56566 Neuwied (DE); ASMUSSEN, Bodo, 56170 Bendorf-Sayn (DE)
(74) Vertreter: Flaccus, Rolf-Dieter
(86) Internationale Anmeldenummer: PCT/EP2000/007897
(87) Internationale Veröffentlichungsnummer: WO 2001/016218

(56) Entgegenhaltungen:
- EP-B- 0 081 913
- WO-A-97/34645
- WO-A-98/01160
- DE-A- 4 201 179
- DE-A- 19 756 314
- DE-A- 19 845 246
- PATENT ABSTRACTS OF JAPAN vol. 13, no. 414 (C-635), 13. September 1989 (1989-09-13) & JP 01 152104 A (HOKKAIDO SODA KK), 14. Juni 1989 (1989-06-14) & DATABASE WPI Week 198930 Derwent Publications Ltd., London, GB; AN 1989-215715

## Beschreibung

Die Erfindung betrifft schnellzerfallende, partikuläre Wirkstoffträger. Sie betrifft insbesondere poröse, wirkstoffhaltige Pellets zur peroralen Applikation auf der Basis von Chitosan oder einem basischen Chitosanderivat. Ferner betrifft sie die Verwendung von Chitosan-Pellets zur Herstellung von Arzneimitteln und Diagnostika.

Partikuläre Wirkstoffträger erfreuen sich einer großen Beliebtheit in der pharmazeutischen Technologie. Für Produkte zur peroralen Applikation haben sie gegenüber flüssigen Darreichungsformen den Vorteil, daß sie leichter und kompakter sind, eine höhere chemische Stabilität aufweisen und exakter zu dosieren sind. Ein Vorteil der multipartikulären Zubereitungen wie Pellets gegenüber "single units" wie Tabletten ist die bessere Reproduzierbarkeit ihres Verhaltens, vor allem wenn sie hochvariablen physiologischen Bedingungen ausgesetzt sind, da sich aufgrund der Mehrzahl der verabreichten Pellets ihr Gesamtverhalten nach statistischen Gesetzmäßigkeiten um einen zu erwartenden Mittelwert bewegt und einzelne Ausreißer nicht in dem Maße zum Tragen kommen, wie es bei einer Tablette der Fall sein kann.

Der Stand der Technik kennt eine große zahl an Trägermaterialien, die sich zur Formung von Pellets eignen. Grundsätzlich handelt es sich um physiologisch unbedenkliche Substanzen mit unterschiedlichen chemischen, physikochemischen und mechanischen Eigenschaften. Die Auswahl hängt im Einzelfall von technischen, ökonomischen und regulatorischen Parametern ab, z. B. von der Kompatibilität des Trägermaterials mit dem oder den Wirkstoffen, von den Zerfalls- und Auflösungseigenschaften, von der Stabilität der Zubereitung, dem Rohstoffpreis, der Verarbeitbarkeit, dem positiven regulatorischen Status für die perorale Anwendung u. s. w.

Neben Pellets für Zubereitungen mit kontrollierter Wirkstofffreisetzung beschreibt der Stand der Technik auch solche mit schnellen Zerfallseigenschaften, die in der Lage sind, ihren enthaltenen Wirkstoff rasch abzugeben. Entsprechende Arzneiformen, auch Akutformen genannt, sind bei sporadisch auftretenden Indikationen, bei denen eine möglichst schnelle pharmakologische Wirkung erwünscht ist, besonders gefragt. Beispiele hierfür sind Analgetika, Antitussiva, Antiallergika, Antiastbmatika, Angina-pektoris-Mittel und andere. Die Trägerstoffe in solchen Zubereitungen sind in der Regel hydrophil bzw. wasserlöslich, um die gewünschten Zerfallseigenschaften zu ermöglichen. Letztere hängen jedoch auch von weiteren Parametern ab, so etwa von dem Vorhandensein von sog. Sprengmitteln, d. h. Substanzen, die in der Lage sind, rasch und unter starker Quellung Wasser aufzunehmen, oder auch von einer möglichst großen wirksamen Oberfläche.

Pellets mit großer äußerer Oberfläche weisen folgerichtig eine kleine Teilchengröße auf. Damit eine Oberfläche wirksam im Sinne der Auflösung ist, muß sie benetzbar sein, was entweder durch die Auswahl des Trägermaterials oder durch den Zusatz von Netzmitteln sichergestellt werden kann. Alternativ hierzu kann eine große Oberfläche auch durch eine hohe Porosität gegeben sein. In diesem Fall spielt der Teilchendurchmesser eine eher untergeordnete Rolle.

DE 42 01 172 Cl beschreibt Pellets, welche als Wirkstoff Aloe Vera-Extrakt enthalten, und in denen als Träger Gelatine oder Kollagen enthalten ist, wobei es sich vorzugsweise um einen kaltwasserlöslichen Typ der Gelatine handelt.

Ein weiterer Trägerstoff, z. B. Dextran, ein Zucker, Zuckeralkohol, Glycin oder Polyvinylpyrrolidon kann ebenfalls enthalten sein. Als Herstellverfahren wird vorgeschlagen, ein Tropfverfahren, z. B. unter Verwendung der in DE 37 11 169 A1 offenbarten Apparatur anzuwenden, bei dem die Pellets durch Erstarren der Tröpfchen in einer Kühlflüssigkeit, vorzugsweise in flüssigem Stickstoff entstehen. Eine anschließende Gefriertrocknung führt zu dem gewünschten Endprodukt, das eine hohe Porosität und Zerfallsgeschwindigkeit aufweisen sollte.

Auch die DE 42 01 173 A1 offenbart derartige Pellets, allerdings enthalten diese ein Dihydropyridinderivat als Wirkstoff.

Diese Cryopellets auf der Basis von Gelatine machen sich die seit langem bekannte Eignung dieses Trägermaterials für die Gefriertrocknung zur Erzeugung poröser Produkte zunutze: Z. B. befinden sich in Deutschland derartige Produkte zur oralen (z. B. Imodium® lingual, gefriergetrocknete Plättchen, Fa. Janssen-Cilag) und parenteralen Anwendung (z. B. Mumpsvax® Trockensubstanz) auf dem Markt.

Diese gelatine- oder kollagenhaltigen Zubereitungen besitzen den Nachteil, daß ihr Erfolg durch die Verunsicherung der Bevölkerung hinsichtlich der Gefahren einer BSE-Kontamination beeinträchtigt wird. Viele Patienten bzw. Ärzte ziehen Produkte ohne Gelatine vor.

DE 42 01 179 A1 offenbart Pellets, bestehend aus hydrophilen Makromolekülen und Wirkstoffen, wobei der Wirkstoff in einer Matrix gelöst, suspendiert oder emulgiert vorliegen kann, sowie die Verwendung dieser Pellets als Arzneimittel oder Diagnostikum. DE 42 01 179 A1 offenbart auch ein Tropfverfahren zur Herstellung wirkstoffhaltiger Pellets, bei dem eine Makromoleküle und Wirkstoff enthaltende Flüssigkeit in eine tiefkalte Flüssigkeit eingetropft wird. Dabei wird angegeben, daß mit Chitosan oder Gelatine Pellets erzeugt werden, die die Eigenschaft haben, den Wirkstoff im Darmsaft verzögert bzw. retardiert freizusetzen.

WO 98/01 160 beschreibt Zusammensetzungen zum Einführen von Genen in Epithelzellen, die im wesentlichen aus einem Komplex aus Chitosan mit Nukleinsäuren bestehen, wobei die Produkte ein Zetapotential von +5 mV bis +60 mV haben. Die mit dem in WO 98/01 160 beschriebenen Verfahren hergestellten Partikel weisen eine Größe von mehr als 1 mm auf.

WO 97/34 645 betrifft beschichtete, biologisch abbaubare poröse Pellets, welche auf Chitosan basieren können, für eine Wundheilung durch langsame, kontrollierte Freisetzung eines Wirkstoffs in der Wunde. Dabei können die beschichteten Chitosanpellets als Wundimplantat verwendet werden und sind auch zwei Wochen nach Implantation noch im Gewebe vorhanden.

Es ist daher die Aufgabe der vorliegenden Erfindung, poröse, schnellzerfallende Pellets bereitzustellen, welche ohne den Einsatz von Gelatine, Kollagen oder deren Derivate auskommen. Eine weitere Aufgabe ist die Bereitstellung von gelatine- und kollagenfreien, porösen, schnellzerfallenden Pellets als Wirkstoffträger für die Herstellung von Arzneimitteln und Diagnostika.

Die Aufgabe wird erfindungsgemäß durch Pellets nach Anspruch 1 gelöst.

Es hat sich herausgestellt, daß sich durch ein Tropfverfahren, bei dem eine wäßrige Dispersion mit Chitosan oder ein basisches Chitosanderivat als Trägersubstanz eingesetzt wird Cryopellets bzw. lyophilisierte Pellets mit vergleichbarer Qualität herstellen lassen wie die in der Literatur beschriebenen gelatinehaltigen Pellets.

Pellets im Sinne dieser Erfindung sind sphärische oder annähernd sphärische feste Gebilde mit einem Durchmesser von ca. 0,3 bis 5 mm. Die Dosiseinheit einer pharmazeutischen Polletzubereitung besteht aus einer Mehrzahl von Pellets. Die Pellets sind schnellzerfallend, d. h. es handelt sich nicht um Pellets mit verzögerter, retardierter, kontrollierter oder modifizierter Freisetzung. Obwohl die Freisetzungsgeschwindigkeit nicht mit der Zerfallsgeschwindigkeit gleichzusetzen ist, stehen die beiden doch im Zusammenhang miteinander, so daß man schnellzerfallende Zubereitungen dann einsetzt, wenn im Sinne einer Akutform auch eine schnelle Wirkstofffreisetzung und ein rascher Wirkungseintritt erwünscht sind. In der Regel zerfallen solche Zubereitungen in physiologischen Flüssigkeiten innerhalb von einigen Minuten. Die Pellets sind zudem porös, d. h. sie weisen eine innere Oberfläche auf, deren Ausmaß im Vergleich zur äußeren Oberfläche nicht vernachlässigbar ist.

Das Herstellverfahren ist durch eine Abfolge von mehreren Schritten gekennzeichnet, die durchaus bei Bedarf vom Fachmann durch weitere Schritte ergänzt werden können, welche vor, zwischen oder im Anschluß an die beschriebenen Verfahrensschritte gestellt werden können.

In einem ersten Schritt wird eine wäßrige Lösung oder Dispersion hergestellt, worin Chitosan oder ein basisches Chitosanderivat, ein oder mehrere Wirkstoffe, ggf. weitere Hilfsstoffe und eine Säure überwiegend gelöst vorliegen, d. h. ihr möglicherweise ungelöster Anteil ist wesentlich kleiner als ihr gelöster Anteil. Dies trifft insbesondere für das als Trägerstoff der Pellets eingesetzte Chitosan oder das basische Chitosanderivat zu; die Verwendung von lediglich suspendiertem Chitosan führt nicht zu geeigneten Pellets mit ausreichender Kohäsion.

Wie fast alle Biopolymere läßt sich Chitosan, welches selbst ein Derivat, nämlich ein partielles Deacetylierungsprodukt des nativen Polymers Chitin darstellt, in vielfältiger Weise derivatisieren und modifizieren, um seine chemischen oder physikochemischen Eigenschaften zu verändern. Ein basisches Chitosanderivat ist ein aus Chitosan durch einen chemischen, biologischen oder physikalischen Modifikationsprozeß hervorgegangenes Polymer, welches jedoch wie Chitosan eine Anzahl an positiven Ladungen besitzt. Durch den Modifikationsprozeß kann die Anzahl der positiven Ladungen kleiner sein als die des Ursprungspolymers; ebenso können durch den Modifikationsprozeß negative Ladungen im Molekül eingeführt worden sein. Im Sinne der Erfindung kann ein beliebiges, physiologisch akzeptables Chitosanderivat verwendet werden, solange es immer noch eine positive Gesamtladung hat oder die Anzahl der positiven Ladungen die Anzahl der negativen Ladungen übersteigt. Bevorzugte Chitosanderivate sind solche, die durch Acylierung des Chitosans entstehen.

Zu den bevorzugten nichtmodifizierten Chitosantypen gehören diejenigen, die eine Molmasse von mehr als 40.000 besitzen; besonders bevorzugt sind solche mit einer Molmasse von mehr als 75.000. Eine bevorzugte Ausführungsform verwendet ferner nichtmodifizierte Chitosane mit einem Acetylierungsgrad von 10 bis 50 %; besonders bevorzugt sind Acetylierungsgrade von 20 bis 45 %.

Der Einsatz von Chitosan oder Chitosanderivaten hat den Vorteil, daß es sich um physiologisch besonders gut verträgliche Biopolymere handelt, die auf einfaches Weise aus dem billigen und in großen Mengen vorhandenen Rohstoff Chitin gewonnen werden können, und bei denen eine BSE-Infektionsgefahr ausgeschlossen werden kann.

Während Chitosan in Wasser selbst weitgehend unlöslich ist, steigt die Löslichkeit bei einer Verschiebung des pH-Werts zum sauren Milieu deutlich an. Um eine nennenswerte Polymerkonzentration zu erhalten, ist es daher erforderlich, die Lösung oder Dispersion unter gleichzeitiger Verwendung einer Säure herzustellen. Um diese später aus den Pellets leichter wieder entfernen zu können, hat sich gezeigt, daß die Säure einen niedrigen Siedepunkt aufweisen sollte, nämlich vorzugsweise max. 140°C, insbesondere max. 120°C, besonders bevorzugt max. 100 °C, ganz besonders bevorzugt max. 80°C, wie Chlorwasserstoff, Bromwasserstoff, Trifluoressigsäure, Ameisensäure und Essigsäure. Geeignet sind auch Säuren, die mit Wasser ein niedriger siedendes binäres Azeotrop bilden, wie Essigsäure oder Propionsäure. Vorzugsweise handelt es sich um eine physiologisch unbedenkliche Säure; es ist jedoch auch denkbar, eine weniger verträgliche Säure einzusetzen, solange sichergestellt ist, daß sie später praktisch vollständig aus den Pellets entfernt wird. Dies ist schwieriger bei Säuren, die im Bereich von Wasser oder höher sieden, da drastischere Trocknungsbedingungen eingesetzt werden müssen, bei denen unter Umständen das Produkt übertrocknet und der Wirkstoff abgebaut wird.

Empfindliche Produkte wird man daher unter vermindertem Druck trocknen oder gefriertrocknen.

Bei dem Wirkstoff handelt es sich um eine Substanz, die verabreicht wird, um im oder am lebenden menschlichen oder tierischen Körper eine pharmakologische Wirkung im weitesten Sinne auszuüben. Darüber hinaus sind in dem Begriff Substanzen eingeschlossen, die zu diagnostischen Zwecken verabreicht werden.

Weitere, dem Fachmann bekannte pharmazeutische Hilfsstoffe können in der Lösung oder Dispersion ebenfalls vorliegen. Bei diesen kann es sich z. B. um weitere polymere oder nichtpolymere Trägerstoffe handeln, aber auch um Stabilisatoren, Tenside, Zerfallsbeschleuniger, Antioxidantien, Farbstoffe, Pigmente, Aromen, Süßstoffe oder sonstige Geschmacksverbesserer, Bindemittel u. s. w.

In einem weiteren Verfahrensschritt wird die wäßrige Lösung oder Dispersion in eine Kühlflüssigkeit mit einer Temperatur von höchstens -5°C eingetropft und dadurch tröpfchenweise zum Erstarren gebracht. Die Erzeugung der Tropfen kann z. B. durch eine pipettenartige Vorrichtung, durch eine Nadel oder durch eine Düse geschehen, durch welche die wäßrige Lösung oder Dispersion gepumpt wird. Beim Fall - in der Regel durch die Luft oder eine Schutzgasphase - nehmen die Tröpfchen eine sphärische Gestalt an, die nach dem Eintauchen in die Kühlflüssigkeit konserviert wird, indem das kugelförmige oder annähernd kugelförmige Tröpfchen zum Erstarren gebracht wird. In Abhängigkeit von verschiedenen, dem Fachmann bekannten Parametern, z. B. der Dichte und viskosität der wäßrigen Phase, der Form, dem Durchmesser und der Grenzflächenspannung an der Abtropfvorrichtung, der Pumpgeschwindigkeit u.s.w., können Tröpfchen mit unterschiedlicher Größe erzeugt werden. Bevorzugt werden solche Ausgestaltungen, bei denen Tröpfchen mit einem Durchmesser von 0,3 bis 5 mm geformt werden. Die Tröpfchengröße bestimmt maßgeblich die Teilchengröße der aus dem Verfahren hervorgehenden Pellets mit, wenn auch die beiden Größen nicht gleichzusetzen sind. In der Regel wird die modale Tröpfchengröße etwas größer als der modale Pelletdurchmesser sein.

Um eine sofortige Erstarrung zu bewirken, muß die Temperatur der Kühlflüssigkeit deutlich unter 0°C liegen und darf im Sinne der Erfindung höchstens -5°C betragen. Bevorzugt ist die Ausführung der Erfindung mit einer Kühlflüssigkeit mit einer Temperatur von weniger als -15°C. Besonders bevorzugt sind Kühlflüssigkeiten, bei denen es sich um tiefkalte, inerte, verflüssigte Gase oder Gasmischungen handelt, z. B. um flüssige Luft oder flüssigen Stickstoff. Bei einer solchen Ausführungsform ist es am ehesten sichergestellt, daß eine unmittelbare Erstarrung der wäßrigen Lösung oder Dispersion beim Eintritt in die Kühlflüssigkeit geschieht. Derartige Kühlmittel lassen sich auch besonders gut und praktisch vollständig aus dem Produkt entfernen.

In einem weiteren Verfahrensschritt werden die erstarrten Tröpfchen, die nunmehr Pellets darstellen, isoliert. Dies kann je nach der Konfiguration der verwendeten Tropf- und Kühlapparatur auf unterschiedliche Weise geschehen. Eine einfache Möglichkeit ist, die pellethaltige Kühlflüssigkeit durch ein Sieb zu geben. Dabei kann gleichzeitig eine Klassierung der Pellets vorgenommen werden. Erfindungsgemäße Pellets, die aus dem beschriebenen Verfahren hervorgehen, weisen eine Teilchengröße von etwa 0,3 bis 5 mm auf. Bevorzugt sind Polletdurchmesser von 0,8 bis 3 mm.

In einem weiteren Verfahrensschritt werden die derart isolierten Pellets getrocknet. Wegen des hohen Wassergehaltes sollte bei der Isolierung und Trocknung unter normalen Druckverhältnissen eine Temperatur von etwa 0°C nicht überschritten werden. Empfehlenswert ist dagegen die Gefriertrocknung unter vermindertem Druck, bei der das Wasser auch bei etwas höheren Temperaturen durch Sublimation aus den Pellets entfernt und eine hochporöse Pelletstruktur erhalten werden kann. Diesbezügliche Apparaturen und Verfahrensparameter sind dem Fachmann bekannt.

Die Erfindung betrifft auch die Pellets, welche aus dem Verfahren hervorgehen. Entsprechend obigen Ausführungen sind diese sphärisch, porös, schnellzerfallend, und haben vorzugsweise eine Teilchengröße von 0,3 bis 5 mm im Durchmesser, besonders bevorzugterweise 0,8 bis 3 mm. Ihre Zusammensetzung ist ferner so gewählt, daß sie in einer bevorzugten Ausführungsform eine Oberflächenladung aufweisen, die sich als Zetapotential von +0,5 bis +50 mV ausdrücken läßt. Diese Oberflächenladung beruht darauf, daß die Pellets im wesentlichen auf dem Trägerstoff Chitosan oder einem basischen Chitosanderivat basieren.

Zur besseren Handhabbarkeit und Applizierbarkeit der Pellets können diese dosisweise in Hartgelatinekapseln oder vergleichbare Hartkapseln aus Stärke oder anderen Polymeren abgefüllt vorliegen. Während Hartgelatinekapseln für die Verabreichung von Pellets üblich sind, kann es aufgrund der o. g. BSE-Problematik, von der die Pellets selbst nicht betroffen sind, angebracht sein, ein anderes Kapselmaterial wie z. B. Stärke zu wählen.

Alternativ zur Verabreichung als Hartkapsel ist auch eine Anwendung als Instantzubereitung möglich. In diesem Fall können die Pellets, in einem Mehrdosenbehälter oder auch dosisweise in Sachets verpackt vorliegend, in Wasser oder eine andere Flüssigkeit eingebracht werden, worin sie zerfallen und eine trinkfertige Zubereitung bilden. Für einen solchen Anwendungszweck, allerdings auch für die Abfüllung in Hartkapseln, kann es notwendig sein, die erfindungsgemäßen Pellets mit weiteren Eilfsstoffen zu mischen, durch welche z. B. ihre Fließfähigkeit, Adhäsionsneigung, Stabilität u.s.w. beeinflußt wird. In diesem Sinne schließt die erfindungsgemäße Verwendung der Pellets jede Art der Weiterverarbeitung zu einem Arzneimittel oder Diagnostikum ein.

## Patentansprüche

1. Poröse, in physiologischen Flüssigkeiten innerhalb von einigen Minuten zerfallende, wirkstoffhaltige Pellet auf Basis von Chitosan oder einem basichen Chitosanderivat, **dadurch gekennzeichnet, daß** die Pellets eine mittlere Teilchengröße von 0,3 bis 5 mm im Durchmesser, ein Zetapotential von +0,5 bis +50 mV aufweisen, und durch ein Verfahren hergestellt wurden, bei dem man
a) eine wäßrige Lösung oder Dispersion herstellt, worin
- Chitosan oder das basische Chitosanderivat,
- ein oder mehrere Wirkstoffe,
- eine Säure, und
- ggf. weitere Hilfsstoffe
überwiegend gelöst vorliegen,
b) die wäßrige Lösung oder Dispersion in eine Kühlflüssigkeit mit einer Temperatur von höchstens -5°C eintropft und **dadurch** tröpfchenweise zum Erstarren bringt,
c) die erstarrten Tröpfchen bzw. Pellets isoliert,
d) trocknet und die Säure aus den Pellets entfernt.

2. Pellets nach Anspruch 1, **dadurch gekennzeichnet, daß** das verwendete Chitosan oder Chitosanderivat eine Molmasse von mehr als 40.000, vorzugsweise von mehr als 75.000 besitzt.

3. Pellets nach Anspruch 1 oder 2, **dadurch gekennzeichnet, daß** das verwendete Chitosan oder Chitosanderivat einen Acetylierungsgrad von 10 bis 50%, vorzugsweise von 20 bis 45% aufweist.

4. Pellets nach einem der vorangehenden Ansprüche, **dadurch gekennzeichnet, daß** es sich bei dem basischen Chitosanderivat um ein acyliertes Chitosan handelt.

5. Pellets nach einem der vorangehenden Ansprüche, **dadurch gekennzeichnet, daß** die Pellets eine mittlere Teilchengröße 0,8 bis 3 mm im Durchmesser aufweisen.

6. Pellets nach einem der vorangehenden Ansprüche, **dadurch gekennzeichnet, daß** die Pellets zur Applikation in einer Hartgelatinekapsel vorliegen.

7. Pellets nach einem der vorangehenden Ansprüche, **dadurch gekennzeichnet, daß** die Pellets vor der Einnahme in eine Flüssigkeit gegeben werden, in welcher sie zerfallen.

8. Pellets nach Anspruch 1, **dadurch gekennzeichnet, daß** die Säure einen Siedepunkt von höchstens 140°C aufweist.

9. Pellets nach Anspruch 1, **dadurch gekennzeichnet, daß** die Trocknung der isolierten Pellets durch eine Gefriertrocknung geschah.

10. Pellets nach Anspruch 1, **dadurch gekennzeichnet, daß** die Kühlflüssigkeit eine Temperatur von weniger als -15°C aufwies.

11. Verwendung von Pellets nach einem der vorangehenden Ansprüche zur Herstellung eines Arzneimittels oder Diagnostikums.

## Claims

1. Porous, active substance-containing pellets disintegrating within a few minutes and being based on chitosan or a basic chitosan derivative, **characterized in that** the pellets have a mean particle size of 0.3 to 5 mm in diameter and a zetapotential of +0.5 to +50 mV, and that they are produced by means of a method wherein
a) an aqueous solution or dispersion is prepared wherein
- chitosan or the basic chitosan derivative,
- one or more active substances,
- an acid, and
- possibly further auxiliary substances
are present, predominantly in solution,
b) the aqueous solution or dispersion is dripped into a cooling liquid having a temperature of maximally -5°C and is thereby solidified in the form of droplets;
c) the solidified droplets or pellets are isolated and
d) dried, and the acid is removed from the pellets.

2. Pellets according to Claim 1, **characterized in that** the chitosan or chitosan derivative used has a molar mass of more than 40,000, preferably more than 75.000.

3. Pellets according to claim 1 or 2, **characterised in that** the chitosan or chitosan derivative used has an acetylation degree of 10 to 50%, preferably 20 to 45 %.

4. Pellets according to any one of the preceding claims, **characterised in that** the basic chitosan derivative is an acylated chitosan.

5. Pellets according to any one of the preceding claims, **characterised in that** said pellets have a mean particle size of 0.8 to 3 mm in diameter.

6. Pellets according to any one of the preceding claims, **characterised in that**, for the purpose of application, said pellets are present in a hard gelatine capsule.

7. Pellets according to any one of the preceding claims, **characterised in that**, prior to intake, said pellets are placed in a liquid in which they disintegrate.

8. Pellets according to claim 1, **characterised in that** the acid has a boiling point of at maximum 140 °C.

9. Pellets according to claim 1, **characterised in that** the drying of the isolated pellets is carried out by means of a freeze-drying process.

10. Pellets according to claim 1, **characterised in that** the cooling liquid has a temperature of less than -15°C.

11. Use of the pellets according to any one of the preceding claims for manufacturing a medicament or a diagnostic agent.

## Revendications

1. Pellet poreux, contenant une ou plusieurs substances actives, se décomposant en quelques minutes dans les liquides physiologiques, à base de chitosane ou d'un dérivé basique du chitosane, **caractérisé en ce que** les pellets présentent une grosseur moyenne des particules de 0,3 à 5 mm en diamètre, un potentiel zêta de +0,5 à +50 mV et sont préparés par un procédé dans lequel
a) on prépare une solution ou une dispersion aqueuse dans laquelle se trouvent, sous forme essentiellement dissoute,
- le chitosane ou le dérivé basique du chitosane,
- une ou plusieurs substances actives,
- un acide, et
- le cas échéant d'autres adjuvants,
b) on introduit goutte à goutte la solution ou la dispersion aqueuse dans un liquide de refroidissement à une température d'au maximum -5°C et on l'amène ainsi à se solidifier par gouttes,
c) on isole les gouttes solidifiées ou pellets,
d) on les sèche et on élimine l'acide des pellets.

2. Pellets selon la revendication 1, **caractérisés en ce que** le chitosane ou le dérivé de chitosane utilisé présente une masse molaire supérieure à 40 000, de préférence supérieure à 75 000.

3. Pellets selon la revendication 1 ou 2, **caractérisés en ce que** le chitosane ou le dérivé de chitosane utilisé présente un degré d'acétylation de 10 à 50%, de préférence de 20 à 45%.

4. Pellets selon l'une quelconque des revendications précédentes, **caractérisés en ce qu'**il s'agit, pour le dérivé basique du chitosane, d'un chitosane acylé.

5. Pellets selon l'une quelconque des revendications précédentes, **caractérisés en ce que** les pellets présentent une grosseur moyenne des particules de 0,8 à 3 mm en diamètre.

6. Pellets selon l'une quelconque des revendications précédentes, **caractérisés en ce que** les pellets se trouvent dans une capsule de gélatine dure pour l'administration.

7. Pellets selon l'une quelconque des revendications précédentes, **caractérisés en ce que** les pellets sont introduits avant la prise dans un liquide dans lequel ils se décomposent.

8. Pellets selon la revendication 1, **caractérisés en ce que** l'acide présente un point d'ébullition d'au maximum 140°C.

9. Pellets selon la revendication 1, **caractérisés en ce que** le séchage des pellets isolés est réalisé par lyophilisation.

10. Pellets selon la revendication 1, **caractérisés en ce que** le liquide de refroidissement présente une température inférieure à -15°C.

11. Utilisation de pellets selon l'une quelconque des revendications précédentes pour la préparation d'un médicament ou d'un agent diagnostique.
